# EUROPEAN PATENT SPECIFICATION

(11) **EP 4 066 871 B1**
(45) Date of publication and mention of the grant of the patent: **08.10.2025**
(21) Application number: 20899541.5
(22) Date of filing: 07.12.2020
(51) Int. Cl.: A61M 1/16

(54) **BLOOD PURIFICATION DEVICE**
BLUTREINIGUNGSVORRICHTUNG
DISPOSITIF DE PURIFICATION DE SANG

(30) Priority: 13.12.2019 JP 2019225071
(43) Date of publication of application: 05.10.2022
(73) Proprietor: Nipro Corporation, Osaka-shi, Osaka 531-8510 (JP)
(72) Inventor: NAGAI, Sho, Osaka-shi, Osaka 531-8510 (JP)
(74) Representative: Prüfer & Partner mbB Patentanwälte · Rechtsanwälte
(86) International application number: PCT/JP2020/045383
(87) International publication number: WO 2021/117654

(56) References cited:
- WO-A2-2014/049560
- JP-A- 2004 049 493
- JP-A- 2018 042 624
- JP-A- H0 910 300
- JP-A- H0 919 497
- US-A1- 2019 201 609

## Description

### TECHNICAL FIELD

The present invention relates to a blood purification device.

### BACKGROUND ART

Japanese Patent Laying-Open No. 2018-42624 (PTL 1) is a prior document that discloses a blood purification device that performs an extracorporeal ultrafiltration method (ECUM).

The blood purification device described in PTL 1 includes a dialyzer, a dialysis fluid introduction line, a dialysis fluid drainage line, and a water removal pump. The dialyzer purifies blood flowing in a blood circuit. Via the dialysis fluid introduction line, a dialysis fluid can be introduced into the dialyzer. Via the dialysis fluid drainage line, a drainage fluid from the dialyzer can be drained. The water removal pump can remove water from the blood flowing in the blood circuit. The blood purification device is configured to perform ultrafiltration treatment by stopping the introduction of the dialysis fluid into the dialyzer and driving the water removal pump for the water removal.

Further relevant prior art is for instance disclosed in PTL2, PTL3 and PTL4.

### CITATION LIST

### PATENT LITERATURE

PTL 1: Japanese Patent Laying-Open No. 2018-42624
PTL2: US2019/201609 A1
PTL3: WO2014/049560 A2
PTL4: JP2004049493 A

### SUMMARY OF INVENTION

### TECHNICAL PROBLEM

With the blood purification device described in PTL 1, the fluid can be replenished during ultrafiltration treatment or blood can be returned immediately after the ultrafiltration treatment, and a timing of switching between the dialysis and the ultrafiltration is not taken into consideration.

The present invention has been made in view of the above problem, and has an object to provide a blood purification device that can remove a set amount of water while suppressing a substance in blood from being removed more than necessary.

### SOLUTION TO PROBLEM

A blood purification device according to the present invention comprises the technical features as defined in independent claim 1. The blood purification device includes a blood purifier, a dialysis fluid tube path, a drainage fluid tube path, a fluid supplying mechanism, a fluid draining mechanism, and a monitoring device. The blood purifier is incorporated in a blood circuit in which blood flows from an upstream side toward a downstream side. In the dialysis fluid tube path, a dialysis fluid is supplied into the blood purifier. In the drainage fluid tube path, a drainage fluid drained from the blood purifier flows. The fluid supplying mechanism is provided in the dialysis fluid tube path to send out the dialysis fluid flowing in the dialysis fluid tube path. The fluid draining mechanism is provided in the drainage fluid tube path to send out the drainage fluid flowing in the drainage fluid tube path. The monitoring device monitors a concentration of a monitoring target component in the drainage fluid flowing in the drainage fluid tube path. Before a water removal amount reaches a set value and when the monitoring device does not detect that the concentration of the monitoring target component is less than or equal to a threshold value, the fluid supplying mechanism and the fluid draining mechanism are driven to perform dialysis and water removal. Before the water removal amount reaches the set value and when the monitoring device detects that the concentration of the monitoring target component is less than or equal to the threshold value, the fluid supplying mechanism is stopped and the fluid draining mechanism is driven to perform the water removal for ultrafiltration. When the water removal amount reaches the set value, the fluid supplying mechanism and the fluid draining mechanism are stopped.

In one embodiment of the present invention, the monitoring target component is urea.

### ADVANTAGEOUS EFFECTS OF INVENTION

According to the present disclosure, a set amount of water can be removed while suppressing a substance in blood from being removed more than necessary.

### BRIEF DESCRIPTION OF DRAWINGS

Fig. 1 is a circuit diagram showing a configuration of a blood purification device according to a first embodiment of the present invention.
Fig. 2 is a flowchart showing an operation of the blood purification device according to one embodiment of the present invention.

### DESCRIPTION OF EMBODIMENTS

Hereinafter, a blood purification device according to one embodiment of the present invention will be described with reference to figures. In the following description of the embodiment, the same or corresponding portions in the figures are denoted by the same reference characters, and will not be described repeatedly. In the following description of the embodiment, a blood purification device used for general hemodialysis (HD) will be described as the blood purification device. However, the blood purification device may be a blood purification device used for any one of hemodiafiltration (HDF), continuous renal replacement therapy (CRRT), continuous hemodiafiltration (CHDF), continuous hemofiltration (CHF), and continuous hemodialysis (CHD), and is preferably used for continuous therapy, apheresis therapy, or the like.

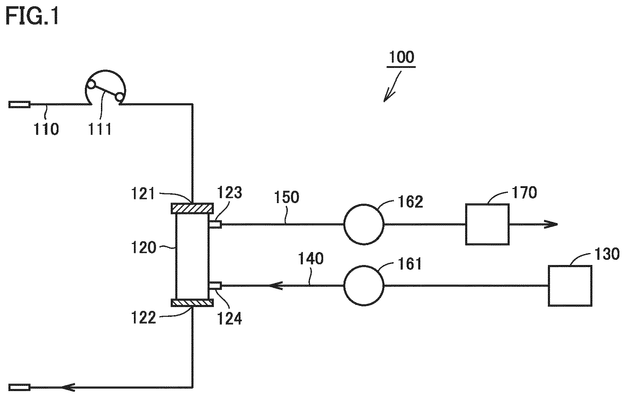

Fig. 1 is a circuit diagram showing a configuration of a blood purification device according to a first embodiment of the present invention. As shown in Fig. 1, a blood purification device 100 according to the first embodiment of the present invention includes a blood purifier 120, a dialysis fluid tube path 140, a drainage fluid tube path 150, a fluid supplying mechanism 161, a fluid draining mechanism 162, and a monitoring device 170. Blood purification device 100 further includes a dialysis fluid supply source 130 and a controller. The controller is electrically connected to each of fluid supplying mechanism 161, fluid draining mechanism 162, and monitoring device 170. The controller controls the operation of each of fluid supplying mechanism 161 and fluid draining mechanism 162 based on a detection result input from monitoring device 170.

Blood purifier 120 includes a semipermeable membrane constituted of, for example, a hollow fiber membrane. Blood purifier 120 has a blood inlet 121 and a blood outlet 122. Blood inlet 121 is connected to the upstream side of blood circuit 110. The downstream side of blood circuit 110 is connected to blood outlet 122.

Blood circuit 110 is provided with a blood pump 111 that sends out blood. Blood flows from the upstream side toward the downstream side of blood circuit 110. Blood purifier 120 is incorporated in blood circuit 110.

Blood purifier 120 further includes a dialysis fluid inlet 124 and a drainage fluid outlet 123. Dialysis fluid tube path 140 is connected to dialysis fluid inlet 124. Drainage fluid tube path 150 is connected to drainage fluid outlet 123.

The upstream end of dialysis fluid tube path 140 is connected to dialysis fluid supply source 130 that supplies the dialysis fluid. Fluid supplying mechanism 161 is connected to dialysis fluid tube path 140 so as to send out the dialysis fluid at a set flow rate Qd. Fluid supplying mechanism 161 is a peristaltic pump, but may be a roller pump. The dialysis fluid flowing in dialysis fluid tube path 140 is supplied into blood purifier 120.

The drainage fluid drained from blood purifier 120 is drained from the downstream end of drainage fluid tube path 150. Fluid draining mechanism 162 is connected to drainage fluid tube path 150 so as to send out, at a set flow rate Qu, the drainage fluid flowing in drainage fluid tube path 150. Fluid draining mechanism 162 is a peristaltic pump, but may be a roller pump.

The configuration of fluid supplying mechanism 161 is not particularly limited as long as the dialysis fluid flowing in dialysis fluid tube path 140 can be sent out.

The configuration of fluid draining mechanism 162 is not particularly limited as long as the drainage fluid flowing in drainage fluid tube path 150 can be sent out. For example, fluid supplying mechanism 161 and fluid draining mechanism 162 may allow a fresh dialysis fluid to flow into blood purifier 120 and allow a spent dialysis fluid to be drained from blood purifier 120 in accordance with a closed volume-controlled system such as a double-chamber system, a viscous system, or a dual-pump system.

In each of these systems, a pump and an on/off valve or check valve are connected to each of dialysis fluid tube path 140 and drainage fluid tube path 150.

Fluid supplying mechanism 161 and fluid draining mechanism 162 in the viscous system may be configured to supply a fresh dialysis fluid and drain a drainage fluid by using a pump to bring silicone oil into and out of two respective viscous rooms of two viscous chambers each including a fresh dialysis fluid room, a drainage fluid room, and the viscous room, for example. Thus, each of fluid supplying mechanism 161 and fluid draining mechanism 162 is driven by at least one of driving of the pump and on/off driving of the on/off valve.

Monitoring device 170 monitors the concentration of a monitoring target component in the drainage fluid flowing in drainage fluid tube path 150. In the present embodiment, the monitoring target component is urea. The monitoring target component is, however, not limited to urea, and may be β2 microglobulin, creatinine, uric acid, or the like.

Monitoring device 170 continuously measures a change in ultraviolet absorbance of the drainage fluid so as to calculate a transition in the concentration of urea in the drainage fluid in accordance with a change in blood urea nitrogen correlated with a change in absorbance, for example. The measurement by monitoring device 170 is not limited to the continuous measurement, and may be measurement that is performed at certain intervals. The target to be measured by monitoring device 170 is not limited to the ultraviolet absorbance, and may be a differential refractive index or the like.

Hereinafter, an operation of blood purification device 100 according to the present embodiment will be described.

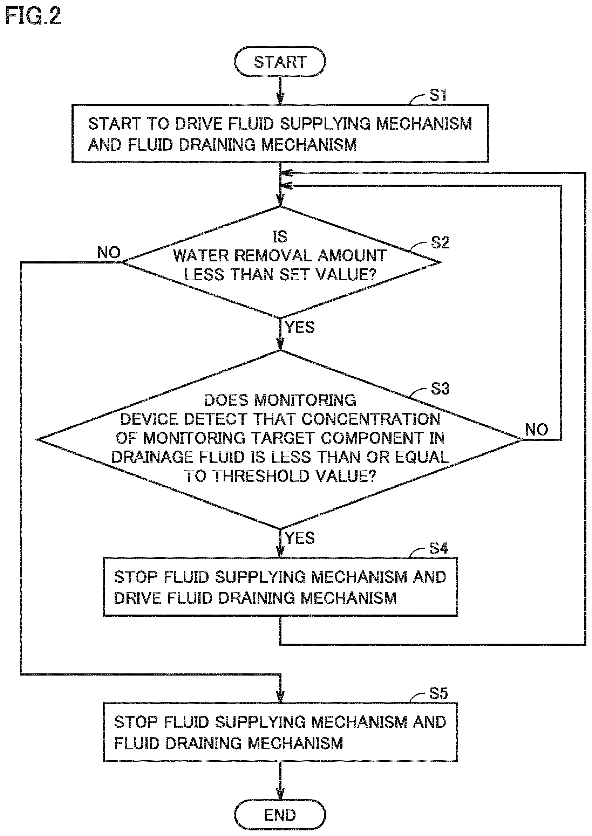

Fig. 2 is a flowchart showing the operation of the blood purification device according to the embodiment of the present invention. First, the controller sets respective threshold values for a water removal amount, a water removal rate, and the concentration of the monitoring target component. The controller determines a flow rate Qd of the dialysis fluid and a flow rate Qu of the drainage fluid to cause the water removal rate to attain the set value. The water removal rate is represented as Qd-Qu.

Next, as shown in Fig. 2, in response to signals from the controller, fluid supplying mechanism 161 starts to be driven to attain set flow rate Qd, and fluid draining mechanism 162 starts to be driven to attain set flow rate Qu (step S1). Further, blood pump 111 starts to be driven. Accordingly, the dialysis fluid is supplied to blood purifier 120, with the result that dialysis and water removal are performed.

Next, the controller determines whether or not the water removal amount is less than the set value (step S2). When the water removal amount is less than the set value, the controller checks whether or not the monitoring device detects that the concentration of the monitoring target component in the drainage fluid is less than or equal to the threshold value (step S3).

When the monitoring device detects that the concentration of the monitoring target component in the drainage fluid is less than or equal to the threshold value, the controller stops fluid supplying mechanism 161 and drives fluid draining mechanism 162 (step S4). In this way, ultrafiltration is performed with the supply of the dialysis fluid to blood purifier 120 being stopped and the water removal being performed. As a result, only the water removal can be performed without diffusion in the blood purifier 120, so that urea, β2 microglobulin, creatinine, and the like in the blood can be suppressed from being removed more than necessary.

When the monitoring device does not detect that the concentration of the monitoring target component in the drainage fluid is less than or equal to the threshold value, the process returns to step S2. After the ultrafiltration is started in step S4, the process returns to step S2.

When the water removal amount reaches the set value, the controller stops fluid supplying mechanism 161 and fluid draining mechanism 162 (step S5). Thus, the set amount of water can be removed.

As described above, in blood purification device 100 according to the present embodiment, before the water removal amount reaches the set value and when monitoring device 170 does not detect that the concentration of the monitoring target component is less than or equal to the threshold value, fluid supplying mechanism 161 and fluid draining mechanism 162 are driven to perform dialysis and water removal. Before the water removal amount reaches the set value and when monitoring device 170 detects that the concentration of the monitoring target component is less than or equal to the threshold value, fluid supplying mechanism 161 is stopped and fluid draining mechanism 162 is driven to perform the water removal for ultrafiltration. When the water removal amount reaches the set value, fluid supplying mechanism 161 and fluid draining mechanism 162 are stopped. This makes it possible to remove a set amount of water while suppressing a substance in blood from being removed more than necessary.

Urea is present substantially evenly in blood due to its small molecular weight, and by setting urea as the monitoring target component, a state of removal of uremic toxin in the blood can be precisely known. By setting the threshold value for the concentration of urea to, for example, 20 mg/dL, the concentration of urea in the blood of a dialysis patient can be maintained at the same level as that of a healthy person.

The embodiments disclosed herein are illustrative and non-restrictive in any respect. The scope of the present invention is defined by the terms of the claims, rather than the embodiments described above.

### REFERENCE SIGNS LIST

100: blood purification device; 110: blood circuit; 111: blood pump; 120: blood purifier; 121: blood inlet; 122: blood outlet; 123: drainage fluid outlet; 124: dialysis fluid inlet; 130: dialysis fluid supply source; 140: dialysis fluid tube path; 150: drainage fluid tube path; 161: fluid supplying mechanism; 162: fluid draining mechanism; 170: monitoring device.

## Claims

1. A blood purification device comprising:
a blood purifier (120) incorporated in a blood circuit (110) in which blood flows from an upstream side toward a downstream side;
a dialysis fluid tube path (140) in which a dialysis fluid is supplied into the blood purifier;
a drainage fluid tube path (150) in which a drainage fluid drained from the blood purifier flows;
a fluid supplying mechanism (161) provided in the dialysis fluid tube path (140) to send out the dialysis fluid flowing in the dialysis fluid tube path;
a fluid draining mechanism (162) provided in the drainage fluid tube path (150) to send out the drainage fluid flowing in the drainage fluid tube path; and
a monitoring device (170) that is configured to monitor a concentration of a monitoring target component in the drainage fluid flowing in the drainage fluid tube path (140), and
a controller electrically connected to each of the fluid supplying mechanism (161), the fluid draining mechanism (162) and the monitoring device (170);
**characterized in that** the controller is configured to:
before a water removal amount reaches a set value and when the monitoring device (170) does not detect that the concentration of the monitoring target component is less than or equal to a threshold value, drive the fluid supplying mechanism (161) and the fluid draining mechanism (162) to perform dialysis and water removal,
before the water removal amount reaches the set value and when the monitoring device (170) detects that the concentration of the monitoring target component is less than or equal to the threshold value, stop the fluid supplying mechanism and drive the fluid draining mechanism to perform the water removal for ultrafiltration, and
when the water removal amount reaches the set value, stop the fluid supplying mechanism (161) and the fluid draining mechanism (162).

2. The blood purification device according to claim 1, wherein the monitoring target component is urea.

## Patentansprüche

1. Blutreinigungsvorrichtung, umfassend:
einen Blutreiniger (120), der in einen Blutkreislauf (110) integriert ist, in dem Blut von einer stromaufwärts gelegenen Seite zu einer stromabwärts gelegenen Seite fließt;
einen Dialysefluid-Rohrweg (140), in dem ein Dialysefluid in den Blutreiniger zugeführt wird;
einen Drainagefluid-Rohrweg (150), in dem ein aus dem Blutreiniger abgeleitetes Drainagefluid fließt;
einen Fluidzufuhrmechanismus (161), der in dem Dialysefluid-Rohrweg (140) vorgesehen ist, um das in dem Dialysefluid-Rohrweg fließende Dialysefluid auszugeben;
einen Fluidableitmechanismus (162), der in dem Drainagefluid-Rohrweg (150) vorgesehen ist, um das in dem Drainagefluid-Rohrweg fließende Drainagefluid auszugeben; und
eine Überwachungsvorrichtung (170), die ausgebildet ist, eine Konzentration einer Überwachungs-Zielkomponente in dem in dem Drainagefluid-Rohrweg (140) fließenden Drainagefluid zu überwachen, und
eine Steuereinrichtung, die elektrisch mit dem Fluidzufuhrmechanismus (161), dem Fluidableitmechanismus (162) und der Überwachungsvorrichtung (170) verbunden ist;
**dadurch gekennzeichnet, dass** die Steuereinrichtung ausgebildet ist, um:
bevor eine Wasserentnahmemenge einen Sollwert erreicht und wenn die Überwachungsvorrichtung (170) nicht erfasst, dass die Konzentration der Überwachungs-Zielkomponente kleiner oder gleich einem Schwellenwert ist, den Fluidzufuhrmechanismus (161) und den Fluidableitmechanismus (162) anzutreiben, um eine Dialyse und eine Wasserentnahme durchzuführen,
bevor die Wasserentnahmemenge den Sollwert erreicht und wenn die Überwachungsvorrichtung (170) erfasst, dass die Konzentration der Überwachungs-Zielkomponente kleiner oder gleich dem Schwellenwert ist, den Fluidzufuhrmechanismus anzuhalten und den Fluidableitmechanismus anzutreiben, um die Wasserentnahme zur Ultrafiltration durchzuführen, und
wenn die Wasserentnahmemenge den Sollwert erreicht, den Fluidzufuhrmechanismus (161) und den Fluidableitmechanismus (162) anzuhalten.

2. Blutreinigungsvorrichtung nach Anspruch 1, wobei die Überwachungs-Zielkomponente Harnstoff ist.

## Revendications

1. Dispositif de purification de sang comprenant :
un purificateur de sang (120) incorporé dans un circuit de sang (110) dans lequel le sang s'écoule d'un côté amont vers un côté aval ;
un trajet de tube de fluide de dialyse (140) dans lequel un fluide de dialyse est fourni dans le purificateur de sang ;
un trajet de tube de fluide de drainage (150) dans lequel un fluide de drainage drainé du purificateur de sang s'écoule ;
un mécanisme d'alimentation en fluide (161) prévu dans le trajet de tube de fluide de dialyse (140) pour envoyer le fluide de dialyse s'écoulant dans le trajet de tube de fluide de dialyse ;
un mécanisme de drainage de fluide (162) prévu dans le trajet de tube de fluide de drainage (150) pour envoyer le fluide de drainage s'écoulant dans le trajet de tube de fluide de drainage ; et
un dispositif de surveillance (170) qui est configuré pour surveiller une concentration d'un composant cible de surveillance dans le fluide de drainage s'écoulant dans le trajet de tube de fluide de drainage (140), et
un dispositif de commande connecté électriquement à chacun du mécanisme d'alimentation en fluide (161), du mécanisme de drainage de fluide (162) et du dispositif de surveillance (170),
**caractérisé en ce que** le dispositif de commande est configuré pour :
avant qu'une quantité d'élimination d'eau n'atteigne une valeur définie et lorsque le dispositif de surveillance (170) ne détecte pas que la concentration du composant cible de surveillance est inférieure ou égale à une valeur seuil, entraîner le mécanisme d'alimentation en fluide (161) et le mécanisme de drainage de fluide (162) pour effectuer une dialyse et une élimination d'eau,
avant que la quantité d'élimination d'eau n'atteigne la valeur définie et lorsque le dispositif de surveillance (170) détecte que la concentration du composant cible de surveillance est inférieure ou égale à la valeur seuil, arrêter le mécanisme d'alimentation en fluide et entraîner le mécanisme de drainage de fluide pour effectuer l'élimination d'eau pour une ultrafiltration, et
lorsque la quantité d'élimination d'eau atteint la valeur définie, arrêter le mécanisme d'alimentation en fluide (161) et le mécanisme de drainage de fluide (162).

2. Dispositif de purification de sang selon la revendication 1, dans lequel le composant cible de surveillance est l'urée.
